# EUROPÄISCHE PATENTANMELDUNG

(11) **EP 1 783 220 A1**
(43) Veröffentlichungstag der Anmeldung: **09.05.2007**
(21) Anmeldenummer: 05024207.2
(22) Anmeldetag: 07.11.2005
(51) Int. Cl.: C12N 15/10

(54) **Verfahren zur selektiven, reversiblen Adsorption von Nukleinsäuren an ein Trägermaterial**

(71) Anmelder: QIAGEN GmbH, 40724 Hilden (DE); Forschungszentrum Jülich GmbH, 52425 Jülich (DE)
(72) Erfinder: Frerix, Andreas, 47546 Kalkar-Kehrum (DE); Hubbuch, Jürgen, Dr., 50937 Köln (DE); Müller, Markus, Dr., 41542 Dormagen (DE); Schönewald, Michael, 24790 Schacht-Audorf (DE)
(74) Vertreter: Leidescher, Thomas

(57) **Zusammenfassung**

Die Erfindung betrifft ein Verfahren zur selektiven, reversiblen Adsorption von Nukleinsäuren an ein Trägermaterial, das dadurch gekennzeichnet ist, daß (a) ein einen kosmotropen Salzgehalt von mindestens 1 Mol aufweisendes, nukleinsäurehaltiges Gemisch mit dem Trägermaterial in Kontakt gebracht wird, woraufhin die Nukleinsäure bzw. die Nukleinsäuren aus dem nukleinsäurehaltigen Gemisch an dem Trägermaterial adsorbiert wird bzw. werden, und (b) die am Trägermaterial gebundenen Nukleinsäure bzw. Nukleinsäuren mittels einer wäßrigen Lösung, welche einen Salzgehalt von nicht mehr als 1 Mol aufweist, von dem Trägermaterial wieder abgelöst wird.

## Beschreibung

Die vorliegende Erfindung betrifft ein Verfahren zur selektiven, reversiblen Adsorption von einer Nukleinsäure oder mehreren Nukleinsäuren an ein Trägermaterial.

Die effiziente Isolierung von Nukleinsäuren aus kosmotropen Lösungen mit hoher Salzkonzentration (kosmotrope Salze sind z.B. Phosphate, Sulfate und Citrate) und insbesondere aus der Unterphase von wäßrigen Zwei-Phasensystemen (Polymer-Salz Systeme) stellt ein bisher praktisch nicht gelöstes Problem bei der Aufreinigung von Nukleinsäuren dar. Angewandt wurden bisher einige zeit-intensive Methoden (z.B. Ultrafiltration, Gelfiltration etc.), die allerdings nur in wenigen Anwendungen zu rechtfertigen sind. Bisher ist eine schnelle, effiziente und kostengünstige Präzipitation von Nukleinsäuren unter derartigen Lösungsbedingungen nicht möglich (z.B. durch Zugabe eines organischen Lösungsmittels, wie Isopropanol), da aufgrund der Nichtmischbarkeit erneut ein Phasensystem ausgebildet wird und Teile der Nukleinsäuren anschließend unvollständig als Präzipitat in der Phasengrenzfläche vorliegen. Darüber hinaus sind Fällungen im allgemeinen kompliziert skalierbare Aufarbeitungstechniken. Nach dem Stand der Technik erfolgt aus diesem Grund eine Umpufferung bzw. Formulierung von nukleinsäurehaltigen Phasen durch Ultrafiltration, Gelfiltration oder anderen geeigneten Methoden basierend auf säulenchromatographischen Verfahren.

Es sind auch Verfahren zur Reinigung von doppelsträngiger DNA (vornehmlich Plasmid-DNA) nach dem so genannten wäßrigen Zweiphasensystem ("aqueous two-phase system", "ATPS") bekannt, das z.B. in WO2004/106516 A1 und Trinidade, I.P., Diogo M.M., Prazeres D.M.F. und Marcos J.C., Purification of plasmid DNA vectors by aqueous two-phase extraction and hydrophobic interaction chromatography, Journal of Chromatography A, 1082 (2005), 176-184, beschrieben ist. Bei letzterem ATPS Verfahren wird die produkthaltige Unterphase mittels konventioneller hydrophober Interaktionschromatographie weiter gereinigt, wobei die Reinigung durch eine selektive Adsorption einer nicht erwünschten Nukleinsäure erreicht wird. Die Produkt-Nukleinsäure wird hierbei nicht gebunden und somit auch keine Konzentrierung oder Isolierung aus der hoch-salzhaltigen Phase erreicht.

Die Nachteile der oben genannten säulenchromatographischen Methoden nach dem Stand der Technik (insbesondere hydrophobe Verfahren) liegen vorwiegend darin, daß sie hohe Kosten verursachen, niedrigere Flußraten und somit eine niedrige Produktivität aufweisen, hohe Druckverluste zeigen, nur geringe Kapazitäten aufweisen sowie Elutionsbedingungen mit hohen Salzkonzentrationen benötigen, die ihrerseits zu Entsorgungsproblemen führen. Da in allen bekannten Fällen RNA stärker an das Adsorbens bindet als DNA, ist eine gleichzeitige Entsalzung und eine Trennung von RNA/DNA nicht möglich. Die Ultra- bzw. Dia-Filtration hat einen hohen Zeitbedarf, eine geringe Produktivität und nur eine geringe Selektivität für die unterschiedlichen Nukleinsäurepopulationen wie Plasmid-DNA, gescheerte genomische DNA und langkettige RNA-Moleküle. Bei der Gelfiltration schließlich ist nachteilig, daß wegen der geringen zu applizierenden Mengen eine hohe Produktverdünnung und eine niedrige Produktivität in Kauf genommen werden müssen.

Es ist daher Aufgabe der vorliegenden Erfindung, ein verbessertes Verfahren zur selektiven Abtrennung von Nukleinsäuren aus wäßrigen, kosmotrope Salze enthaltenden Systemen, wie z.B. Zwei-Phasen-Systemen, zur Verfügung zu stellen. Diese Aufgabe löst die Erfindung durch das Verfahren gemäß des unabhängigen Anspruchs 1. Weitere vorteilhafte Ausgestaltungen, Aspekte und Details der Erfindung ergeben sich aus den abhängigen Ansprüchen, der Beschreibung, dem Beispiel und der Zeichnung.

Die vorliegende Erfindung beschreibt somit ein Verfahren zur selektiven, reversiblen Adsorption von Nukleinsäuren an ein Trägermaterial, das dadurch gekennzeichnet ist, daß (a) ein einen kosmotropen Salzgehalt von mindestens 1 Mol aufweisendes, nukleinsäurehaltiges Gemisch mit dem Trägermaterial in Kontakt gebracht wird, woraufhin die Nukleinsäure bzw. die Nukleinsäuren aus dem nukleinsäurehaltigen Gemisch an dem Trägermaterial adsorbiert wird bzw. werden, und (b) Ablösen der am Trägermaterial gebundenen Nukleinsäure bzw. Nukleinsäuren mittels einer wäßrigen Lösung, welche einen Salzgehalt von nicht mehr als 1 mol, insbesondere weniger als 1 mol, aufweist. Bevorzugt wird zum Ablösen der Nukleinsäure von dem Trägermaterial (oben Schritt (b)) eine Lösung eingesetzt, die eine Salzkonzentration von 500 mM oder weniger, z.B. 100 mM oder weniger, 50 mM oder weniger, 20 mM oder weniger bzw. 10 mM oder weniger aufweist. Es ist des weiteren bevorzugt, daß die Lösung zum Ablösen einer Nukleinsäure von dem Trägermaterial eine Salzkonzentration von mindestens 1 mM aufweist. Der Gehalt der Lösung an kosmotropen Salzen in Schritt (a), in dem Nukleinsäure(n) an das Trägermaterial gebunden wird/werden, beträgt vorzugsweise mindestens 1,5 Mol, mehr bevorzugt mindestens 2,0 Mol, und insbesondere mindestens 2,5 Mol. Kosmotrope Salzgehalte von mehr als 5 Mol und insbesondere von mehr als 10 Mol sind weniger bevorzugt.

Kosmotrope (bzw. lyotrope) Salze sind in der sogenannten Hofmeister-Serie nach ihrer Eigenschaft geordnet, die Struktur des Wassers zu erhöhen. Demgegenüber stehen chaotrope Salze, die eine Verringerung (Störung) der Wasserstruktur bewirken. Zu den bekannten kosmotropen Salzen gehören z.B. Phosphate, Sulfate und Citrate.

Die hier beschriebene Art der Adsorption nutzt hydrophobe oder gemischt hydrophobeelektrostatische Wechselwirkungen (sog. "mixed-mode"). Die Stärke dieser Bindung - d.h. das synergistische Wechselspiel zwischen Oberflächeneigenschaften des Adsorbers, des Adsorbens und der Flüssigphasenbedingungen - bestimmt die im Verfahren gewünschte Reversibilität der Bindung. Sie unterscheidet sich somit von der im sogenannten Boom-Patent (U.S. Patent Nr. 5,234,809, Europäisches Patent Nr. 0 389 063) beschriebenen Bindung von Nukleinsäuren unter chaotropen Bedingungen an Silika-Oberflächen.

Weiterhin unterscheidet sich die Bindungsart deutlich von gängigen exklusiv hydrophoben Bindungsarten, die in der Literatur beschrieben sind, da sich im vorliegenden Fall die Adsorptionsreihenfolge von DNA und RNA umkehrt, d.h. daß DNA (als Zielnukleinsäure) stärker bindet als RNA. Dies ermöglicht eine bessere Auslastung der Adsorbentien mit DNA, eine initiale Elution von RNA und eine finale Elution von DNA unter gewünschten Pufferbedingungen mit geringen Salzkonzentrationen. Daher ist eine gleichzeitige Reinigung und Entsalzung der Probe möglich. Durch Kombination bzw. Zugabe von organischen Lösungsmitteln wie Ethanol oder Isopropanol können Bindungs-Selektivitäten weiterhin moduliert werden.

Unter "Zielnukleinsäure" bzw. "Zielnukleinsäuren" werden in Zusammenhang mit der vorliegenden Anmeldung eine Nukleinsäure bzw. mehrere Nukleinsäuren verstanden, die aus einem Gemisch verschiedener Substanzen, z.B. den Komponenten einer Zelle, abgetrennt werden sollen.

Besonders günstig ist der erfindungsgemäße Einsatz dieser Technologie zum Abfangen ("capturing") von Ziel-Nukleinsäuren aus vorher gewonnenen, produkthaltigen Phasen von Polymer/Salz-Phasensystemen geeignet. In der Regel ist eine schnelle, effiziente und kostengünstige Präzipitation der Nukleinsäure(n) aus diesen Lösungsbedingungen nicht möglich (z.B. durch Zugabe von Isopropanol), da sich dann zwei Phasen, bestehend aus der organischen und der nukleinsäurehaltigen Hochsalzphase, ausbilden oder Nukleinsäuren an der Phasengrenzfläche präzipitieren, siehe oben.

Die vorliegende Erfindung betrifft somit ein Verfahren zur selektiven, reversiblen Adsorption von Nukleinsäure(n) aus Lösungen mit einer hohen Konzentration an vorzugsweise kosmotropen Salzen ("hohe Konzentration" bedeutet hier insbesondere ≥ 20% w/w z.B. an Phosphat- Citrat- und Sulfatsalzen) an ein geeignetes Trägermaterial (Adsorbentien, Membranen, Filter oder Monolithe) z.B. unter hydrophoben oder "mixed-mode" (hydrophobelektrostatischen Bedingungen und nachfolgende Desorption der Nukleinsäure(n) von dem Trägermaterial und Elution durch Formulierungslösungen mit niedriger Salzkonzentration von 100 mM oder geringer, vorzugsweise 50 mM oder geringer und insbesondere 20 mM oder geringer (z.B. dem Fachmann bekannte konventionelle wäßrige Puffersysteme wie 10 mM Tris/HCl, 1 mM EDTA, pH 8.0 etc.). Die erfindungsgemäß fixierten Nukleinsäuren können unter gewünschten wäßrigen bzw. nicht-kosmotropen und/oder Niedrigsalz-Bedingungen von der Adsorber-Matrix eluiert werden und in nachfolgenden Applikationen eingesetzt werden. Die erfindungsgemäße Adsorption des Nukleinsäure-Zielmoleküls erfolgt aus hochmolaren Lösungen kosmotroper Salze an geeignete Adsorbentien, Membranen, Filter oder Monolithe, wie z.B. Nitrocellulose- und hydrophilisierte Nylonfilter, Nylonwatte und Polyvinylidenfluorid (PVDF) sowie auch Adsorbentien auf Silika-Basis. Besonders geeignet ist der erfindungsgemäße Einsatz dieser Technologie zum Abfangen ("capturing") von Nukleinsäuren aus produkthaltigen Phasen von Phasensystemen, in welchen sich die Ziel-Nukleinsäure in der Phase mit hoher Konzentration an kosmotropen Salzen verteilt hat, wie z.B. in der WO2004/106516 A1 beschrieben.

Durch das erfindungsgemäße Verfahren werden die Nachteile des Standes der Technik (wie eine zeitintensive Konzentrierung und Umpufferung der Nukleinsäure-Phase durch z.B. Ultrafiltration oder Gelfiltration; außerdem ist eine Fällung in der Regel aus Lösungen mit hohen Konzentrationen an kosmotropen Salzen nicht möglich) überwunden und die Reinigung von Nukleinsäure(n) erheblich erleichtert. Außerdem ist eine Fällung in der Regel aus Lösungen mit hohen Konzentrationen an kosmotropen Salzen ansonsten i.d. Regel nicht möglich Neben der Umpufferung ermöglicht der erfindungsgemäße Ansatz einen zusätzlichen Aufreinigungseffekt gegenüber Restkontaminanten in der Nukleinsäure-Phase bzw. DNA-Phase (z.B. Proteine, RNA). Das erfindungsgemäße Verfahren eröffnet Anwendungen sowohl im Forschungsmaßstab als auch bei automatisierten Hochdurchsatz-Analysen (z.B. in der Diagnostik) und bei präparativen Anwendungen im Produktionsmaßstab. Weiterhin ermöglicht die Verwendung geeigneter Trägermaterialien (z.B. Adsorbentien, Membranen, Filtern oder Monolithen) den Einsatz hoher Durchflußraten und geringe Verweilzeiten, da der Hauptanteil der Adsorption nicht diffusionslimitiert ist.

Bei dem erfindungsgemäßen Verfahren werden nukleinsäurehaltige Lösungen bevorzugt mit Phosphatkonzentration von mindestens 20 % (w/w) oder nukleinsäurehaltige, kosmotrope, hoch-molare Sulfat- oder Citrat-Lösungen eingesetzt .Bei niedrigeren Konzentrationen dieser kosmotropen Salze ist die Bindung der Nukleinsäuren an das Trägermaterial (Adsorbens) unvollständig. Weiterhin kann man durch Zugabe von nicht-kosmotropen Salzen die notwendige Endkonzentration der bindungsinduzierenden, kosmotropen Salzkomponente modellieren bzw. für die jeweilige Ziel-Nukleinsäure optimieren.

Bei dem Einsatz der vorliegenden Erfindung zur Isolierung von Nukleinsäure(n) aus wäßrigen Zwei-Phasensystemen wird die Unterphase (wie z.B. in WO2004/106516 A1 beschrieben) eingesetzt. Die vorliegende Erfindung eignet sich auch zur Isolierung von Nukleinsäuren aus anderen beschriebenen PEG/Salz-Phasensystemen in Kombination mit kosmotropen Salzen wie Phosphat-, Sulfat- und Citratsalzen, wodurch auch bei diesen die Ergebnisse einer Isolierung nach dem bisherigen Stand der Technik (z.B. durch Ultrafiltration oder Gelfiltration) deutlich verbessert werden.

Die Bindung unter den oben beschriebenen kosmotropen Bedingungen ist besonders effizient und mit höchsten Kapazitäten (bis zu 0,1 mg/cm² Filterfläche und höher) möglich an geeignete Träger wie z.B. Nitrocellulosefilter und hydrophilisierte Nylonfiltermembranen (Membranscheiben, Filterplatten, Filterkapsulen, Filterkartuschen). An PVDF kann ebenfalls nach Spülen mit Isopropanol adsorbiert werden, jedoch mit geringerer dynamischer Kapazität. Andere hydrophobe Membranen wie GHP oder nicht-modifiziertes Nylon führen zwar zur Bindung von Nukleinsäuren, jedoch kann gebundene DNA nicht wie bei Nitrocellulose, PVDF oder hydrophilisiertem Nylon mit wäßrigen Puffern wieder vollständig abgelöst bzw. eluiert werden.

Für die Bindung eignen sich alle nukleinsäurebindenden Adsorbentien, d.h. neben Membranen und Filtermaterialien auch Monolithe, Harze ("Adsorberresins"), Nitrocellulosewatte, Granulate und andere Formate mit adsorbierenden Eigenschaften. Besonders eignen sich jedoch Materialien (Membran, Filter, Monolithe), die eine hohe Produktivität (hohe Durchlaufgeschwindigkeiten, geringe Diffusionslimitierung) aufweisen.

Zur Entfernung von Salzresten kann das Trägermaterial/Adsorbens mit einem Alkohol, insbesondere Isopropanol oder Ethanol, gewaschen werden, vorzugsweise 50 bis 100 % (v/v) Alkohol bzw. Isopropanol oder Ethanol, besonders bevorzugt 60 bis 90 % (v/v) Alkohol bzw. Isopropanol oder Ethanol, ganz besonders bevorzugt 65 bis 85 % (v/v) Alkohol bzw. Isopropanol oder Ethanol. Die Nukleinsäuren liegen dann gefällt auf dem Adsorbens vor.

Die gewaschene, entsalzte, auf der Membran befindliche Nukleinsäure (z.B. DNA) kann in wäßrigen, z.B gepufferten, Lösungen wie Tris/HCl-EDTA (TE), die nicht zur Fällung von DNA führen und zudem keine sehr hohen Konzentrationen (nicht über 1 M) an kosmotropen Salzen, wie Sulfate, Citrate und Phosphate, enthalten, P1 Puffer (QIAGEN GmbH, Hilden Deutschland) oder P3 Puffer (QIAGEN GmbH, Hilden Deutschland), von dem Trägermaterial abgelöst und eluiert werden.

Der Vorteil des erfindungsgemäßen Verfahrens liegt in einer zeitlich deutlich beschleunigten, kostengünstigen, skalierbaren und automatisierbaren Entsalzung, Reinigung, Konzentrierung und/oder Umpufferung von nukleinsäurehaltigen Lösungen, ohne daß aufwendige und limitierte Techniken wie Ultrafiltration, Gelfiltration etc. eingesetzt werden müssen. Dabei kann je nach Zielsetzung und Optimierung der Bedingungen in Abhängigkeit von der jeweiligen Ziel-Nukleinsäure (z.B. Plasmid-DNA, genomische DNA etc.) auch eine deutliche Reduktion von RNA sowie von Proteinen erreicht werden. Die bisher bekannten Verfahren nach dem Stand der Technik werden somit deutlich verbessert.

Das erfindungsgemäße Verfahren läßt sich wie folgt durchführen. Zunächst muß in einem ersten Schritt eine nukleinsäurehaltige Lösung bereitgestellt werden. Diese Lösung kann z.B. durch Ausschütteln von nukleinsäurehaltigen (z.B. DNA-haltigen bzw. plasmidhaltigen) Prozess-Flüssigkeiten, wie Bakterienlysaten, oder durch Aufreinigungstechniken, wie Chromatographie, Präzipitation, Filtration etc., vorgereinigte Prozeß-Zwischenstufen) in einem geeigneten Phasensystem mit einer geeigneten kosmotropen Phase mit hoher Salzkonzentration, wie z.B. in der WO2004/106516 A1 beschrieben, gewonnen werden. Die nukleinsäurehaltige (DNA-haltige bzw. plasmidhaltige Phase) wird dann mittels Adsorption an einem geeigneten Trägermaterial immobilisiert.

Zur Entfernung von Salzresten kann in einem optionalen Schritt das Trägermaterial mit organischen Lösungsmitteln, wie z.B. Alkoholen wie Isopropanol oder Ethanol, gewaschen werden. Alternativ können die Nukleinsäuren auf dem Trägermaterial selektiv auch mit anderen Lösungen fixiert werden, die zur Fällung von Nukleinsäuren führen (z.B. PEG, Spermidin, Spermin, CTAB= Cetyltrimetylammoniumbromid).

Die gewaschene, entsalzte, auf der Membran befindliche Nukleinsäure (z.B. DNA bzw. Plasmid) kann dann in einem weiteren Schritt in wäßrigen, z.B. gepufferten Lösungen, wie TE, die nicht zur Fällung von DNA führen und zudem keine sehr hohen Konzentrationen (nicht mehr als 1 M) kosmotroper Salze wie Sulfate, Citrate und Phosphate enthalten, in P1 Puffer (QIAGEN GmbH) oder in P3 Puffer (QIAGEN GmbH) von dem Trägermaterial abgelöst und eluiert werden.

Die so eluierte(n), gereinigte(n) Nukleinsäure(n) steht/stehen für Analysen, wie z.B. in der Diagnostik, für weitere Aufarbeitungsschritte oder für therapeutische Anwendungen, z.B. gentherapeutische oder genetische Impfungen, zur Verfügung.

Die vorliegende Erfindung befaßt sich mit der selektiven, reversiblen Adsorption von Nukleinsäuren unter kosmotropen Bindungsbedingungen an geeignete Adsorbentien, z.B. an hydrophobe Oberflächen. Besonders bevorzugt sind Nitrocellulose- und Nylonfilter sowie PVDF als Trägermaterialien bzw. Adsorbentien. Besonders bevorzugt ist die selektive, reversible Adsorption von Nukleinsäuren unter kosmotropen Bindungsbedingungen nach Trennung und Extraktion in Phasensystemen aus nukleinsäurehaltigen Phasen von Phasensystemen, insbesondere wäßrigen Zweiphasensystemen, wie sie aus der WO2004/106516 A1 und Trinidade, I.P. et al. (siehe oben) bekannt sind.

Besonders geeignet ist das erfindungsgemäße Verfahren zur Isolierung von doppelsträngigen Nukleinsäuren aus wäßrigen Zwei-Phasensystemen oder organisch/wäßrigen Zwei-Phasensystemen unter kosmotropen Bindungsbedingungen nach Trennung und Extraktion in Phasensystemen aus produkthaltigen Phasen von Phasensystemen an hydrophoben Adsorbentien, wie z.B. Nitrocellulose- und Nylonfiltern und PVDF.

Ein weiterer Aspekt der Erfindung betrifft die Isolierung von doppelsträngigen Nukleinsäuren, wie z.B. Plasmid-DNA, aus der Unterphase eines PEG/Kaliumphosphat-Systems an hydrophoben Adsorbentien, wie z.B. Nitrocellulose- und Nylonfiltern und PVDF.

Die Erfindung wird nachfolgend anhand eines Beispiels näher erläutert.

### Beispiel

300 g eines wäßrigen Zweiphasensystems enthaltend 6 g Biomasse wurden wie in der WO 2004/106516 A1 beschrieben behandelt. Dem Ansatz wurden 15 % Polyethylenglycol (PEG) 800 sowie 20 % Phosphat zugesetzt und die sich einstellende wäßrige Unterphase isoliert. Die Unterphase wurde über eine Nitrocellulosemembran geschickt, und verschiedene Chargen anschließend durch Hochdruckflüssigkeitschromatographie (HPLC) mittels Hydrophober Interaktions-Chromatographie (HIC) analysiert.

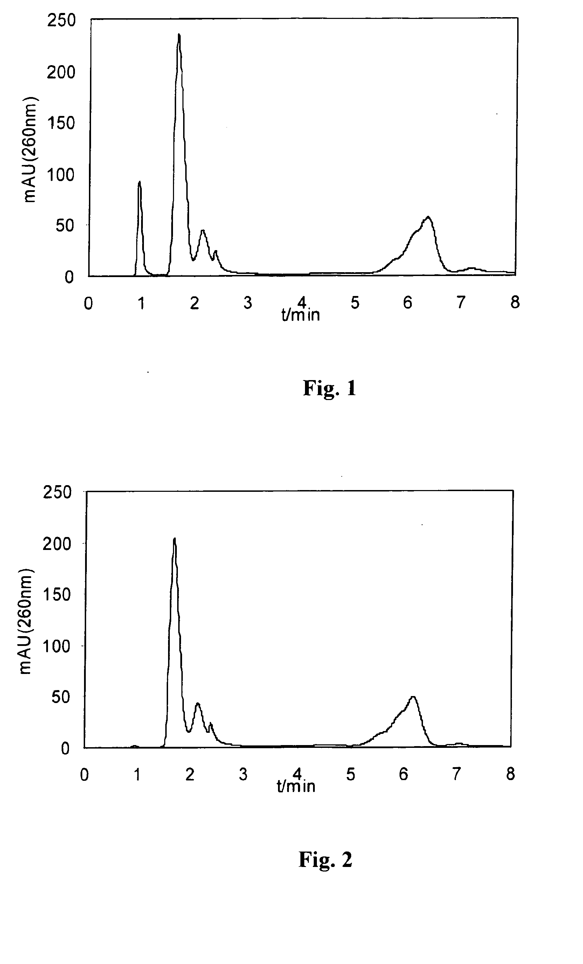

Die Fig. 1 bis 3 zeigen Elutionsprofile von drei HPLC-Läufen. Auf der Ordinate ist die optische Dichte der jeweiligen Probe bei 260 nm aufgetragen, und auf der Abszisse die Zeit in Minuten.

In Fig. 1 ist ein HPLC-Diagramm (Säule: Source 15 PHE (GE Healthcare), Elution mit 1.5 M Ammoniumsulfat, 10 mM Tris/Cl, pH 8.0; 0,8 ml/min nach 2,5 min Gradient zu 10 mM Tris/Cl, pH 8.0 bis zur Elution von RNA) einer Probe gezeigt, welche die Unterphase als Gesamtfraktion, wie nach der wäßrigen Zweiphasentrennung erhalten, darstellt. Die Probe enthielt 26,5 µg/ml pDNA und 57,5 µg/ml RNA. Der erste Peak des in Fig. 1 gezeigten Elutionsprofils bei etwa 1 min geht auf die in der Probe enthaltene pDNA zurück, die nächsten Peaks nach einer Retentionszeit zwischen 1,5 und 2,5 Minuten zeigen Salze, Proteine und Oligonukleotid-RNA, und unter dem letzten Peak, der zwischen 6 und 7 Minuten von der HPLC-Säule eluiert wurde, verbirgt sich RNA.

Fig. 2 zeigt das HPLC-Elutionsprofil der Unterphase, nachdem 20 ml der Unterphase durch eine einen Durchmesser von 47 mm und eine Porengröße von 0,2 µm aufweisende Nitrocellulosemembran filtriert worden waren (30 % w/w Kaliumphosphatpuffer). Die Probe enthielt 0,5 µg/ml pDNA und 50 µg/ml RNA. Wie man leicht erkennen kann, ist die Probe frei von pDNA (kein entsprechender Peak bei etwa 1 min), die praktisch quantitativ an der Nitrocellulosemembran gebunden ist. Die anderen Peaks aus Fig. 1 (Salze, Proteine, Ologonukleotid-RNA, RNA) sind auch in Fig. 2 unverändert vorhanden. Dies zeigt, daß pDNA selektiv und quantitativ aus der Probe entfernt wurde.

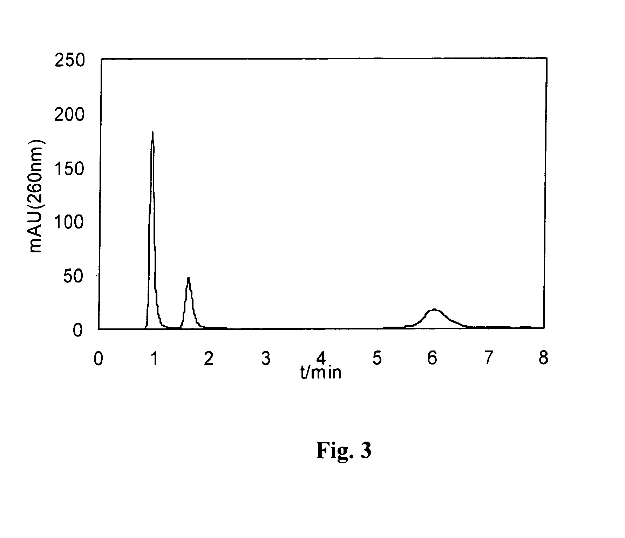

Fig. 3 schließlich zeigt eine Probe, die nach Elution des Nitrocellulosefilters mit 10 ml TE-Puffer (10 mM Tris/HCl, 1 mM EDTA, pH 8,0) erhalten wurde. Die Ausbeute an pDNA betrug 50 µg/ml (entsprechend ca. 95 % Ausbeute; von 530 µg eingesetzter pDNA konnten 501 µg wiedergewonnen werden) und die Menge an RNA in der Probe lag bei 16,4 µg/ml RNA (was einer Abreicherung von ca. 86 % entspricht; 1150 µg RNA wurden eingesetzt, 164 µg fanden sich im Eluat wieder). Damit ist gezeigt, daß pDNA nach einer entsprechenden Behandlung (Bindung an ein Trägermaterial, im vorliegenden Fall ein Nitrocellulosefilter) fast vollständig in hochgereinigter Form wiedergewonnen werden kann.

## Patentansprüche

1. Verfahren zur selektiven, reversiblen Adsorption von Nukleinsäuren an ein Trägermaterial, **dadurch gekennzeichnet, daß**
(a) ein einen kosmotropen Salzgehalt von mindestens 1 Mol aufweisendes, nukleinsäurehaltiges Gemisch mit dem Trägermaterial in Kontakt gebracht wird, woraufhin die Nukleinsäure bzw. die Nukleinsäuren aus dem nukleinsäurehaltigen Gemisch an dem Trägermaterial adsorbiert wird bzw. werden, und
(b) Ablösen der am Trägermaterial gebundenen Nukleinsäure bzw. Nukleinsäuren mittels einer wäßrigen Lösung, welche einen Salzgehalt von nicht mehr als 1 mol aufweist.

2. Verfahren nach Anspruch 1 oder 2, **dadurch gekennzeichnet, daß** das Trägermaterial eine Membran, ein Filter, Nylonwatte oder Polyvinylidenfluorid (PVDF) ist.

3. Verfahren nach Anspruch 2, **dadurch gekennzeichnet, daß** der Filter ein Nitrocellulosefilter oder ein hydrophilisierter Nylonfilter ist.

4. Verfahren nach Anspruche 1 oder 2, **dadurch gekennzeichnet, daß** das Adsorbens aus Silika-Oberflächen besteht.

5. Verfahren nach einem der vorhergehenden Ansprüche, **dadurch gekennzeichnet, daß** in Schritt (a) eine mehr als 1 molare Lösung eines kosmotropen Salzes eingesetzt wird.

6. Verfahren nach Anspruch 5, **dadurch gekennzeichnet, daß** das kosmotrope Salz ein Sulfat, Citrat oder Phosphat ist.

7. Verfahren nach einem der vorhergehenden Ansprüche, **dadurch gekennzeichnet, daß** in Schritt (b) die wäßrigen Lösung, welche einen Salzgehalt von nicht mehr als 1 mol aufweist, ein TE-Puffer (Tris/HCl-EDTA-Puffer) oder Wasser ist.

8. Verfahren nach Anspruch 7, **dadurch gekennzeichnet, daß** der TE-Puffer 10 mM Tris/HCl und 1 mM EDTA enthält und einen pH-Wert zwischen 7 und 8 aufweist.

9. Verfahren nach einem der vorhergehenden Ansprüche, **dadurch gekennzeichnet, daß** nach Schritt (a) und vor Schritt (b) noch ein Reinigungsschritt mit einem organischen Lösungsmittel oder mit einem Fällungsmittel für Nukleinsäuren, insbesondere Spermidin, Spermin, CTAB, PEG, zwischengeschaltet ist.

10. Verfahren nach Anspruch 9, **dadurch gekennzeichnet, daß** das organische Lösungsmittel ein Alkohol, vorzugsweise Ethanol oder Isopropanol, ist.

11. Verfahren nach einem der vorhergehenden Ansprüche, **dadurch gekennzeichnet, daß** es nach einer wäßrigen Zweiphasentrennung von Nukleinsäuren durchgeführt wird.
